# FASCICULE DE BREVET EUROPEEN

(11) **EP 1 098 631 B1**
(45) Date de publication et mention de la délivrance du brevet: **26.03.2003**
(21) Numéro de dépôt: 99929503.3
(22) Date de dépôt: 19.07.1999
(51) Int. Cl.: A61K 9/06, A61K 35/78, A61K 31/7048

(54) **COMPOSITION PHARMACEUTIQUE DESTINEE NOTAMMENT A LA PREVENTION ET AU TRAITEMENT DES RADIOMUCITES ET DES CHIMIOMUCITES**
ARZNEIMITTEL INSBESONDERE ZUR VORBEUGUNG UND BEHANDLUNG VON STRAHLUNGS- UND CHEMOMUKOSITIDEN
PHARMACEUTICAL COMPOSITION IN PARTICULAR FOR PREVENTING AND TREATING MUCOSITIS INDUCED BY RADIOTHERAPY OR CHEMOTHERAPY

(30) Priorité: 20.07.1998 FR 9809230
(43) Date de publication de la demande: 16.05.2001
(73) Titulaire: LABORATOIRE L. LAFON, 94701 Maisons Alfort (FR)
(72) Inventeur: BESSE, Jérôme, Galenix Developpement-Europarc, 33600 Pessac (FR); NGUYEN, Tham, Laboratoire L. Lafon, 94701 Maisons Alfort (FR); LEYDER, Jo[lle, Laboratoire L. Lafon, 94701 Maisons Alfort (FR)
(74) Mandataire: Bernasconi, Jean Raymond
(86) Numéro de dépôt international: FR9901760
(87) Numéro de publication internationale: WO00004878

(56) Documents cités:
- EP-A- 0 380 367
- EP-A- 0 386 960
- EP-A- 0 577 143
- EP-A- 0 648 496
- WO-A-93/21905
- US-A- 5 281 196
- US-A- 5 858 371

## Description

La présente invention concerne une composition pharmaceutique destinée notamment à la prévention et au traitement des radiomucites et des mucites induites par les polychimiothérapies anti-cancéreuses..

A partir des données collectées au cours de la période 1987-1992 parmi ses pays membres, l'Organisation Mondiale de la Santé (OMS) a calculé (pour l'année 1994) une estimation de l'incidence des cancers, selon le sexe, à l'échelle de la planète (World Health Organization : World Health Statistics Annuals, 1987-1992 - Geneva, Switzerland, WHO) : chez l'homme, la localisation caractérisée par la plus grande incidence est la prostate (32 %) ; chez la femme, l'incidence la plus élevée est le cancer du sein (32 %). Chez l'homme, les cancers de la tête et du cou ainsi que de la cavité oro-pharyngée ont une incidence voisine de 6% et l'incidence des cancers colo-rectaux est de 12 %. Chez la femme, l'incidence des cancers "tête et cou, cavité oro-pharyngée" est de 5 % et celle des cancers colo-rectaux de 13 % tandis que l'incidence des cancers utérins est de 8 %. Ces chiffres parlent d'eux-mêmes et montrent d'emblée l'ampleur du problème posé par la prise en compte des effets secondaires des traitements anti-mitotiques mis en jeu, en particulier la radiothérapie et les polychimiothérapies anti-prolifératives.

Selon leurs localisations, la thérapie des cancers fait fréquemment appel à la radiothérapie à moyenne ou haute énergie soit comme traitement de première intention, soit comme thérapeutique adjuvante de la chirurgie et de la chimiothérapie. La radiothérapie est, en particulier, très utilisée pour le traitement de certaines localisations : tête et cou ; cerveau ; cavité oro-pharyngée ; oesophage et estomac ; gros intestin et rectum ; utérus. En 1994, l'incidence des nouveaux cas de cancers dans ces localisations a été estimée par le National Cancer Institute (NCl), pour la population des Etats-Unis :
- tête et cou, cerveau : 17 500 nouveaux cas
- cavité oro-pharyngée : 29 600 nouveaux cas
- larynx: 12 500 nouveaux cas
- oesophage et estomac : 35 000 nouveaux cas
- colon et rectum : 150 000 nouveaux cas
- utérus : 46 000 nouveaux cas.

Grâce aux progrès de la scanno-tomographie, la détermination des champs d'irradiation, la cinétique d'irradiation ainsi que les débits des doses de rayonnements font l'objet d'améliorations régulières. C'est ainsi que pour les cancers "tête et cou", on sait aujourd'hui que le délai entre l'exérèse chirurgicale et la radiothérapie ne doit pas excéder 6 semaines et que toute interruption de la radiothérapie - même en cas d'effets adverses sévères - est préjudiciable à son efficacité. Plus encore, on sait que certaines tumeurs nécessitent une accélération de la radiothérapie (intensification de dose) afin d'atteindre plus efficacement un plus grand nombre de cellules tumorales lorsque celles-ci sont en phase de multiplication : c'est la radiothérapie hyperfractionnée. Dans le même esprit, la recherche constante d'une potentialisation de l'effet thérapeutique a conduit à l'évaluation de la radiochimiothérapie alternée et à la protonthérapie qui permet une focalisation très fine de l'irradiation.

L'irradiation par radiothérapie d'un cancer de l'oesophage ou du larynx induit l'apparition d'une dysphagie douloureuse, source d'une gêne fonctionnelle intense (pouvant générer un amaigrissement important), par agression de la muqueuse par les radiations ionisantes. De même, l'irradiation des tumeurs ou des adénopathies abdominales induit des complications au niveau gastrique. Les nausées et les vomissements sont les manifestations les plus fréquentes. Cependant, peuvent apparaître des altérations épithéliales précoces et surtout des ulcérations douloureuses, souvent très sévères, pouvant persister après la fin du cycle de radiothérapie.

Néanmoins, ce sont les complications buccales de la radiothérapie cervico-faciale qui sont les plus typiques. L'initiation de ce traitement est marquée par une réaction muqueuse plus ou moins intense - la radiomucite oropharyngée - qui s'apparente à un érythème cutané très sévère, consécutif à une brûlure grave induite par une exposition prolongée au rayonnement ultra-violet intense d'origine solaire (saison estivale très chaude ou pays tropicaux). La spécificité de la radiomucite, en particulier oro-pharyngée, est liée à la spécificité de la muqueuse et à sa fragilité. Contrairement aux téguments cutanés qui sont des tissus de revêtement épais, les muqueuses (buccale, gingivale, gastrique, intestinale, utérine, vaginale et ano-rectale) sont très fragiles car constituées de structures cellulaires dépourvues de kératine, très riches en eau et en vaisseaux sanguins. Au sein de tels tissus, l'agitation moléculaire induite par des radiations de haute énergie entraîne une désorganisation extrêmement rapide de l'organisation cellulaire qui est à l'origine de la destruction de la muqueuse. Au contraire du tissu cutané, ces muqueuses n'ont pas de résistance aux agressions de ce type et ne disposent d'aucun système de protection physiologique (ex. : caractère lipo-hydrophile ; vitesse de renouvellement, ...) efficace contre les dégâts occasionnés par l'énergie reçue lors de chaque cycle d'irradiation.

Les conséquences les plus délétères des mucites oro-pharyngées sont la gêne fonctionnelle dont la perception peut être extrêmement variable d'un patient à l'autre, cette gêne n'étant pas reliée à l'intensité de la symptomatologie clinique. La radiomucite peut donc être très invalidante, en particulier lorsque l'érythème est suivi d'un oedème puis d'érosions de la muqueuse pouvant, en plus des algies intenses, gêner gravement l'alimentation.

En outre, l'irradiation des glandes salivaires, prises dans le volume-cible, entraîne une sécheresse de la bouche, souvent intense et durable, voire définitive. Outre l'inconfort de l'hyposialie ou de la xérostomie (privation de salive), qui peut être elle aussi extrêmement mal ressentie, des polycaries peuvent se développer rapidement. A ce stade, le risque majeur des lésions dentaires, au-delà de l'édentation, est de nécessiter l'extraction de la dent sur un os irradié avec constitution d'une ostéoradionécrose, essentiellement mandibulaire. Ainsi, les complications de la xérostomie post-radique sont les mycoses, les infections microbiennes répétées, les polycaries et les ostéoradionécroses et celles-ci sont fréquentes, en particulier, lors des radiothérapies des voies aérodigestives supérieures.

Parce que la mucite peut être aggravée par plusieurs co-facteurs (ex. ; chimiothérapie associée [5-FU, cisplatine], tabagisme, alcoolisme, mauvaise hygiène bucco-dentaire, ...) les risques induits par l'apparition des radiomucites peuvent être d'une extrême gravité. Ils justifient donc la recherche de moyens de prévention efficace de la réaction muqueuse érythémateuse provoquée par les radiations ionisantes.

Les auteurs de la présente invention se sont intéressés à cette question parce que les moyens thérapeutiques actuels de prévention ou de traitement des radiomucites ne sont pas optimisés. En effet, ceux-ci font appel, pour l'essentiel, à l'administration simultanée d'antalgiques (ex. : aspirine), d'antifongiques (ex. : amphotéricine B, miconazole), d'un anesthésique de contact (ex. : xylocaïne) et de bains de bouche (à base de chlorhexidine ou d'hexamidine) systématiquement répétés.

C'est ainsi qu'est née l'idée de concevoir une composition liquide à température ambiante, mais capable d'adhérer à une muqueuse en raison de son passage à l'état gélifié lorsque la température atteint la température de la muqueuse et contenant des substances à activité anti-radicalaire, tout en n'interférant pas avec l'énergie émise par chaque dose de radiothérapie. Conçu pour prévenir l'apparition des mucites bucco-pharyngées consécutives à la radiothérapie des cancers "tête et cou", ce concept de préparation pharmaceutique spécifiquement adapté peut également être appliqué à d'autres mucites induites par la radiothérapie et/ou la chimiothérapie ou bien encore la radiochimiothérapie combinée dans le traitement des cancers tels ceux du colon, du rectum et de l'anus ou lorsque ces thérapeutiques atteignent, de manière incidente, la muqueuse vaginale.

La publication EP-A-0648496 décrit des compositions contenant un flavonoïde et un agent gélifiant.

La présente invention a ainsi pour objet une composition pharmaceutique destinée notamment à la prévention et au traitement des radiomucites et des chimiomucites, comprenant une quantité efficace d'un composé choisi parmi les flavonoïdes et les isoflavonoïdes en mélange avec un véhicule qui est liquide à température ambiante et se gélifie à la température de la muqueuse et qui est capable d'adhérer à la muqueuse en raison de son état gélifié.

Le composé à activité antiradicalaire peut être notamment choisi parmi :

### 1 - les flavonoïdes d'origine naturelle, par exemple :

### i) des flavonols ou flavonolols, parmi lesquels :

- un rutoside : la rutine (quercétine 3-O rutinoside), la quercitrine (quercétine 3-O-rhamnoside), l'isoquercitrine (quercétine 3-O-glucoside),
- la diosmine (diosmétine 7β-rutinoside), l'astragaline (kaempférol 3-O-glucoside), le kaempférol 3-O-rutinoside, la myricitrine (ou myricétine 3-O rhamnoside),
- la robinine (ou kaempférol 3-O-robinoside 7-rhamnoside),
- la kaempféritrine (ou kaempférol 3,7-O dirhamnoside),
- la nobilétine,
- la tangérétine.

### ii) des flavones, parmi lesquelles :

- la rhoifoline (ou apigénine 7-O-néohespéridoside), la lutéoline 7-O glucoside,
- la scutéllarine (ou scutéllaréine 5-O glucoside),
- la pectolinarine (ou pectolinarigénine 7-O rutoside),
- la galutéoline (ou lutéoline 5-O glucoside),
- l'acaciine (ou acacétine 7-O rhamnoglucoside).

### iii) des flavanones, parmi lesquelles :

- la liquiritine (ou liquiritine 4'-O glucoside), la naringine (ou naringénine 7-O néohespéridoside), l'hespéridine (ou hespérétine 7-O-rutinoside).
- l'ériodictine (ou ériodictiol 7-O-rhamnoside)

### 2 - les isoflavonoïdes d'origine naturelle, par exemple :

- la formononétine 7-O glucoside (ou ononine), l'afromosine 7-O glucoside (ou wistine),
- la génistine (ou génistéïne 7-O glucoside), la daidzine, la glycitine,
- la génistéïne 6-O malonylglucoside, la daidzéine 6-O malonylglucoside, la génistéïne 6-O acétylglucoside,
- l'iridine (ou irigénine 7-O-glucoside),
- l'irisolone,
- la tectoridine (ou tectorigénine 7-O-glucoside) ou shékanine.

Le véhicule liquide à température ambiante et qui se gélifie à la température de la muqueuse peut être constitué notamment par une solution ou une dispersion aqueuse d'un mélange de :
a- 0,05 à 5% en poids (de préférence de 0,1 à 3% en poids) d'un agent conférant de la viscosité ;
b - 1 à 20% en poids (de préférence de 5 à 20% en poids) d'un agent modifiant la viscosité en fonction de la température.

i) Les agents conférant de la viscosité peuvent être choisis notamment parmi les composés suivants :
   - colloïdes et hydrocolloïdes (substances polysaccharidiques et apparentées) :
      - galactomannanes et dérivés: gomme guar, gomme de caroube, gomme de tara,...
      - amidon et dérivés
      - gomme arabique, gomme adragante, gomme karaya, ...
      - pectines et dérivés de la pectine,...
      - alginates: acide alginique, alginate de sodium, alginate de sodium/calcium,...
      - carraghénanes et dérivés...
      - cellulose et dérivés : carboxyméthylcellulose (CMC), carboxyméthycellulose sodique, CMC calcique, méthylcellulose, hydroxypropylcellulose, hydroxypropylméthylcellulose, hydroxyéthyl cellulose...
      - dextrans de haut poids moléculaire,
      - xanthanes et dérivés,...
      - acide hyaluronique et dérivés, chitine/chitosan et leurs dérivés...
   - polymères des acides acrylique, méthacrylique et dérivés : polyméthacrylate, polymère carboxyvinylique (carbopol, carbomer) carbophile, polyhydroxyéthyl méthacrylate,
   - dérivés polyvinyliques: polyvinylpyrrolidone, poly(vinylpyrrolidone et vinyl acétate), polyvinylacétatephtalate, alcool polyvinylique,
   - polyéthylène glycols de haut poids moléculaire,
   - polyacrylamide et dérivés,
   - polymères de l'acide maléique, comme par exemple: copolymère de polyvinyléther/acide maléique, sels de sodium/calcium du complexe copolymère de polyvinylether/acide maléique,
   - polystyrène sulfonate de sodium,
   - dérivés minéraux: silice et dérivés silicates, silicones,...
ii) Comme exemple d'agents modifiant la viscosité en fonction de la température, on peut citer :
   - les poloxamères (ex : poloxamère 188, poloxamère 407,...) et les poloxamines,
   - les composés de type divinylbenzène sorbitol (disorbène), solubles en milieu lipophile.

On préfère des compositions qui ont une viscosité inférieure à 200.10⁻³ Pa.s à la température ambiante (20° C) et une viscosité supérieure à 2000.10⁻³ Pa.s à 35-37° C, la viscosité étant déterminée avec un viscosimètre Brookfield de type LV/mobile LV4/vitesse de rotation 0,5 tr/min/lecture après 15 secondes.

A titre d'exemple, une solution selon l'invention qui contient une concentration d'agent conférant de la viscosité - c = 1,7 % en hydroxypropylméthylcellulose (HPMC), - avec 5 % de rutine et 14 % de poloxamère 407, présente le comportement suivant à l'élévation de température :

| **Température** **(t°C)** | **Viscosité *(10***^{***-3***} ***Pa.s)*** |
|---|---|
| **25** | **314** |
| **30** | **1 433** |
| **35** | **3 027** |

Ainsi, à 25° C, la solution est fluide (viscosité de l'ordre de 300.10⁻³ Pa.s) et la gélification est obtenue par passage à une température de 30° C, puis 35° C (la viscosité est multipliée par 10 entre 25 et 35° C).

Les compositions aqueuses ont préférentiellement des pH compatibles avec les muqueuses (en général entre pH 7 et 8).

La présente invention a également pour objet des compositions sous forme solide destinées à être mélangées avec de l'eau pour former une solution liquide à température ambiante et capable de former un gel au contact de la muqueuse à protéger. Pour la muqueuse gastrique et/ou la muqueuse intestinale, on peut ainsi avoir des formes solides telles qu'une poudre ou un granulé, ou bien des granules qui donnent, par addition à un véhicule liquide, une composition liquide (exemple: poudre pour sirop, pour suspension ou solution buvable à préparation extemporanée). Les compositions peuvent également se présenter sous forme de granules ou de comprimés nus à dissoudre dans l'eau juste avant emploi.

Les compositions selon l'invention peuvent contenir d'autres principes actifs associés aux composés à activité antiradicalaire et notamment ceux appartenant aux familles pharmaco-thérapeutiques suivantes :
- antalgiques et antispasmodiques (paracétamol, aspirine, codéine, morphine, atropine, lopéramide, phloroglucinol ...), anesthésiques (xylocaïne, lidocaïne) et antiseptiques (chlorhexidine, hexamidine),
- anti-inflammatoires appartenant à la famille des corticoïdes (prednisolone, triamcinolone, ...) ou des oxicams (ex. : piroxicam, ...),
- anti-ulcéreux (antihistaminiques H₂, prostaglandines et dérivés, inhibiteurs de la pompe à protons tels l'oméprazole, le pantoprazole, le lanzoprazole),
- anti-acides et pansements gastro-intestinaux (phosphate d'aluminium, hydroxyde d'aluminium et de magnésium, argiles (diosmectites, actapulgites ...),
- médicaments du reflux gastro-oesophagien et de la motricité digestive (alginate de sodium, bicarbonate de sodium, métoclopramide ...),
- antiémétiques (benzamides, antihistaminiques H₁, sétrons, ...)
- antidiarrhéiques (lopéramide,...)
- antifongiques à visée digestive (amphotéricine B, nystatine, tioconazole, itraconazole, éconazole, butoconazole....),
- médicaments des troubles fonctionnels digestifs (ex : cisapride) et du transit intestinal,
- anti-bactériens intestinaux (aminosides, nitroimidazoles, polymyxines ...) et anti-viraux (ex: acyclovir),
- produits reconnus pour leurs propriétés adoucissantes et/ou cicatrisantes tels : biotine, polyphénols, acide glycyrrhizinique, thymol, eucalyptol, .., et extraits de plantes riches en acide glycyrrhétinique, pantothénol, allantoïne et dérivés,
- vitamines : du groupe B (B1, B6, B12), nicotinamide, biotine, acide pantothénique,
- produits corrigeant l'hyposialie et régulant la sécrétion salivaire: pilocarpine, anétholtrithione,
- peptides et enzymes : élastine, collagène, glutathion, catalase, endonucléase, pouvant contribuer à la réparation des tissus lésés par l'irradiation.

Les exemples suivants illustrent la présente invention.

### I - Compositions pour la muqueuse buccale

Sans être limitatifs, et pour illustrer l'invention, les préparations suivantes peuvent être présentées en exemples :

| | Pourcentages | | | |
|---|---|---|---|---|
| Exemples | 1 | 2 | 3 | 4 |
| Rutoside hydrosoluble | 2 à 10 | 2 à 10 | 2 à 10 | 2 à 10 |
| Pilocarpine chlorhydrate | --- | 1 à 5 | --- | 1 à 5 |
| Poloxamère 407 | 14,0 | 5 à 20 | 5 à 20 | 5 à 20 |
| HPMC | 1 à 3 | 1 à 3 | 1 à 3 | 1 à 3 |
| Arôme | 0,1 - 0,5 | 0,1 à 0,5 | 0,1 - 0,5 | 0,1 à 0,5 |
| Alpha tocophérol | --- | --- | 0,01 à 0,05 | 0,01 à 0,05 |
| Tampon pH 7,8 QSP | 100 | 100 | 100 | 100 |

Ces compositions constituent des solutions de consistance thermoréversible : fluides à la température ambiante (20°- 25°C), visqueuses à la température (35 - 37° C) des cavités physiologiques. Ainsi, la viscosité à température ambiante (25°C), d'une composition associant 5 à 20 % de poloxamère 407 et 1 à 3 % de polymère HPMC (soit de 6 à 23 % d'agents gélifiants) pourra être suffisamment basse (150 à 300.10⁻³ Pa.s) pour permettre une propulsion aisée (par le système de délivrance) puis une gélification efficace sur la muqueuse à protéger (par passage de la viscosité à 2000 - 21000.10⁻³ Pa.s lorsque la température augmente entre 30 et 35°C, par exemple).

### Il - Composition pour la muqueuse digestive

### 1 - Composition liquide gélifiable

Comme exemples non limitatifs, on peut citer :

| | Pourcentages | | | |
|---|---|---|---|---|
| Exemples | 5 | 6 | 7 | 8 |
| Rutoside | 2 à 10 | 1 à 5 | 0 à 5 | 0 à 5 |
| Amphotéricine B | --- | 1 à 2,5 | --- | --- |
| Miconazole | --- | --- | 1 à 5 | --- |
| Allantoïne | 0 à 1 | 0 à 1 | 0 à 1 | --- |
| Biotine | 0 à 0,050 | 0 à 0,050 | 0 à 0,050 | 0 à 0,050 |
| Dexpanthenol | 0 à 1 | 0 à 1 | 0 à 1 | 0 à 1 |
| Millepertuis | | | | |
| (extrait aqueux) | --- | --- | --- | 0 à 5 |
| Kallanchoe | | | | |
| (extrait aqueux) | --- | --- | --- | 0 à 5 |
| HPMC (Methocel E4M) | 1 à 3 | 1 à 3 | 1 à 3 | 1 à 3 |
| Poloxamère 407 (Lutrol F127) | 6 à 20 | 6 à 20 | 6 à 20 | 6 à 20 |
| Edulcorant/arôme | QS | QS | QS | QS |
| Conservateurs | QS | QS | QS | QS |
| Eau QSP | 100 | 100 | 100 | 100 |

### 2 - Granulés à disperser dans l'eau

A la température du tractus gastro-intestinal, cette composition forme un gel adhérant aux villosités de la muqueuse.

| | (mg) | | | |
|---|---|---|---|---|
| Exemples | 9 | 10 | 11 | 12 |
| Diosmine | 500 | 500 | 500 | 500 |
| Extrait de Centella asiatica | --- | 20 à 50 | --- | --- |
| Hydroxypropylméthyl | | | | |
| cellulose (HPMC) | 150 | 150 | 150 | 150 |
| Gomme xanthane | 250 | 250 | 250 | 250 |
| Carbonate de calcium | 1000 | 1000 | 500 | --- |
| Aldioxa* | --- | --- | 900 | --- |
| Alcloxa** | --- | --- | 100 | --- |
| Poloxamère 407 | 1500 | 1500 | 1500 | 1500 |
| Hydroxyde d'aluminium | --- | --- | --- | 400 |
| Hydroxyde de magnésium | --- | --- | --- | 400 |
| Arôme | QS | QS | QS | QS |
| Xylitol | 1000 | 1000 | 1000 | 1000 |

| | | | | |
|---|---|---|---|---|
| * allantoïnate de dihydroxyaluminium | | | | |
| ** allantoïnate de chlorhydroxylaluminium (pour un sachet à disperser dans un volume de 100 à 200 ml d'eau). | | | | |

### III - Composition pour la muqueuse rectale

Deux exemples de solutions visqueuses thermogélifiables prêtes à l'emploi sont donnés ci-dessous :

| Exemples | 14 (en %) | 15 (en %) |
|---|---|---|
| Rutosides | 2 à 10 | 1 à 5 |
| Dexpanthénol | --- | 1 à 5 |
| Butylhydroxytoluène | --- | 1 à 10 |
| Alpha tocophérol | --- | 0,01 à 0,05 |
| (HPMC) Méthocel E 4M | 1 à 3 | 1 à 3 |
| Poloxamère 407 | 5 à 20 | 5 à 20 |
| Eau purifiée QSP | 100 | 100 |

### IV - Compositions pour la muqueuse vaginale

Trois exemples, non limitatifs, de solutions se gélifiant à la température de la muqueuse sont donnés ci-après :

| Exemples | 16 (en %) | 17 (en %) | 18 (en %) |
|---|---|---|---|
| Rutosides | 0,5 à 10 | 0,5 à 10 | 0,5 à 10 |
| Butoconazole nitrate | 1 à 5 | --- | --- |
| Econazole nitrate | --- | 1 à 3 | --- |
| Thioconazole | --- | --- | 2 à 5 |
| Poloxamère 407 | 6 à 20 | 6 à 20 | 6 à 20 |
| Méthocel E 4M | 1 à 2 | 1 à 2 | 1 à 2 |
| Eau purifiée QSP | 100 | 100 | 100 |

## Revendications

1. Composition pharmaceutique destinée à adhérer à une muqueuse notamment pour la prévention et le traitement des radiomucites, et des chimiomucites induites par la radiothérapie et la radiochimiothérapie combinée, comprenant une quantité efficace d'un composé choisi parmi les flavonoïdes et les isoflavonoïdes en mélange avec un véhicule qui est liquide à température ambiante et se gélifie à la température de la muqueuse et qui est capable d'adhérer à la muqueuse en raison de son état gélifié.

2. Composition selon la revendication 1 dont le véhicule est un véhicule aqueux et comprend un mélange de 0,05 à 5% (de préférence 0,1 à 3%) en poids d'un agent conférant de la viscosité et de 1 à 20% (de préférence 5 à 20%) en poids d'un agent modifiant la viscosité en fonction de la température.

3. Composition selon la revendication 2, dans laquelle l'agent modifiant la viscosité en fonction de la température est choisi parmi les poloxamères, les poloxamines, et les composés divinylbenzène sorbitol.

4. Composition selon la revendication 1, dans laquelle le flavonoïde est choisi parmi les rutosides, la diosmine, la quercitrine, la tangérétine et l'hespéridine.

5. Composition selon la revendication 1, dans laquelle l'isoflavonoïde est la génistine, la daidzine ou la glycitine.

6. Composition selon la revendication 4, dans laquelle le rutoside est la rutine.

7. Composition sous forme solide et formant une composition selon l'une quelconque des revendications 1 à 6 par mélange avec de l'eau.

8. Utilisation d'un composé choisi parmi les flavonoïdes et les isoflavonoïdes en mélange avec un véhicule qui est liquide à température ambiante et se gélifie à la température d'une muqueuse et est capable d'adhérer à cette muqueuse en raison de sa consistance gélifiée, pour la fabrication d'une composition pharmaceutique destinée à la prévention et au traitement des radiomucites et des chimiomucites.

9. Utilisation selon la revendication 8, dans laquelle la composition pharmaceutique est destinée à la prévention et au traitement des radiomucites et des chimiomucites induites par la radiothérapie et la radiochimiothérapie combinée.

10. Utilisation selon la revendication 9, dans laquelle la composition pharmaceutique est destinée à la prévention et au traitement des radiomucites gingivales et oropharyngées.

11. Utilisation selon la revendication 9, dans laquelle la composition pharmaceutique est destinée à la prévention et au traitement des radiomucites anorectales.

12. Utilisation selon la revendication 9, dans laquelle la composition pharmaceutique est destinée à la prévention et au traitement des radiomucites vaginales.

## Claims

1. Pharmaceutical composition intended to adhere to a mucous membrane, particularly for the prevention and treatment of radiation mucitis and chemomucitis induced by radiation therapy and combined radiochemotherapy, comprising an effective amount of a compound selected from among the flavonoids and isoflavonoids mixed with a carrier which is liquid at ambient temperature and gelatinises at the temperature of the mucosa and which is capable of adhering to the mucosa by virtue of its gelatinised state.

2. Composition according to claim 1 wherein the carrier is an aqueous carrier and comprises a mixture of 0.05 to 5% (preferably 0.1 to 3%) by weight of an agent which imparts viscosity and 1 to 20% (preferably 5 to 20%) by weight of an agent which modifies viscosity as a function of temperature.

3. Composition according to claim 2, wherein the agent which modifies viscosity as a function of temperature is selected from among the poloxamers, the poloxamines and the divinylbenzene sorbitol compounds.

4. Composition according to claim 1, wherein the flavonoid is selected from among the rutosides, diosmin, quercitrin, tangeretin and hesperidin.

5. Composition according to claim 1, wherein the isoflavonoid is genistin, daidzih or glycitin.

6. Composition according to claim 4, wherein the rutoside is rutin.

7. Composition in solid form which forms a composition according to any one of claims 1 to 6 by mixing with water.

8. Use of a compound selected from among the flavonoids and isoflavonoids in admixture with a carrier which is liquid at ambient temperature and gelatinises at the temperature of a mucous membrane and is capable of adhering to this mucous membrane by reason of its gelatinised consistency, in order to produce a pharmaceutical composition intended for the prevention and treatment of radiation mucitis and chemomucitis.

9. Use according to claim 8, wherein the pharmaceutical composition is intended for the prevention and treatment of radiation mucitis and chemomucitis induced by radiotherapy and combined radiochemotherapy.

10. Use according to claim 9, wherein the pharmaceutical composition is intended for the prevention and treatment of gingival and oropharyngeal radiation mucitis.

11. Use according to claim 9, wherein the pharmaceutical composition is intended for the prevention and treatment of anorectal radiation mucitis.

12. Use according to claim 9, wherein the pharmaceutical composition is intended for the prevention and treatment of vaginal radiation mucitis.

## Patentansprüche

1. Pharmazeutische Zusammensetzung, die vorgesehen ist, an einer Schleimhaut, insbesondere zur Verhütung und Behandlung von Radiomukositiden und Chemomukositiden, die durch Strahlentherapie und kombinierte Radiochemotherapie hervorgerufen worden sind, anzuhaften, welche eine wirksame Menge einer aus Flavonoiden und Isoflavonoiden ausgewählten Verbindung, gemischt mit einem Träger, der bei Umgebungstemperatur flüssig ist, bei der Temperatur der Schleimhaut geliert und in der Lage ist, auf Grund seines geiförmigen Zustands an dieser anzuhaften, umfasst.

2. Zusammensetzung nach Anspruch 1, deren Träger wässrig ist und ein Gemisch aus 0,05 bis 5 Gew.% (vorzugsweise 0,1 bis 3 Gew.%) eines zähflüssig machenden Mittels und 1 bis 20 Gew.% (vorzugsweise 5 bis 20 Gew.%) eines die Viskosität in Abhängigkeit von der Temperatur modifizierenden Mittels enthält.

3. Zusammensetzung nach Anspruch 2, in welcher das die Viskosität in Abhängigkeit von der Temperatur modifizierende Mittel aus Poloxameren, Poloxaminen und Divinylbenzolsorbit-Verbindungen ausgewählt ist.

4. Zusammensetzung nach Anspruch 1, in welcher das Flavonoid aus Rutosiden, Diosmin, Quercitrin, Tangeretin und Hesperidin ausgewählt ist.

5. Zusammensetzung nach Anspruch 1, in welcher das Isoflavonoid Genistin, Daidzin oder Glycitin ist.

6. Zusammensetzung nach Anspruch 4, in welcher das Rutosid Rutin ist.

7. Zusammensetzung, die in fester Form vorliegt und durch Vermischen mit Wasser eine Zusammensetzung nach einem der Ansprüche 1 bis 6 bildet.

8. Verwendung einer aus Flavonoiden und Isoflavonoiden ausgewählten Verbindung, gemischt mit einem Träger, der bei Umgebungstemperatur flüssig ist, bei der Temperatur einer Schleimhaut geliert und in der Lage ist, auf Grund seiner gelförmigen Konsistenz an dieser anzuhaften, zur Herstellung einer pharmazeutischen Zusammensetzung, die zur Verhütung und Behandlung von Radiomukositiden und Chemomukositiden vorgesehen ist.

9. Verwendung nach Anspruch 8, bei welcher die pharmazeutische Zusammensetzung zur Verhütung und Behandlung von Radiomukositiden und Chemomukositiden, die durch Strahlentherapie und kombinierte Radiochemotherapie hervorgerufen worden sind, vorgesehen ist.

10. Verwendung nach Anspruch 9, bei welcher die pharmazeutische Zusammensetzung zur Verhütung und Behandlung von Radiomukositiden des Zahnfleischs und des Mundrachenraums vorgesehen ist.

11. Verwendung nach Anspruch 9, bei welcher die pharmazeutische Susammensetzung zur Verhütung und Behandlung anorektaler Radiomukositiden vorgesehen ist.

12. Verwendung nach Anspruch 9, bei welcher die pharmazeutische Zusammensetzung zur Verhütung und Behandlung vaginaler Radiomukositiden vorgesehen ist.
